# EUROPEAN PATENT SPECIFICATION

(11) **EP 0 837 702 B1**
(45) Date of publication and mention of the grant of the patent: **03.03.2004**
(21) Application number: 95928270.8
(22) Date of filing: 07.06.1996
(51) Int. Cl.: A61M 1/00

(54) **DRAINAGE UNIT WITH CONTROLLED NEGATIVITY RELIEF FEATURE**
DRAINAGEEINHEIT MIT GEREGELTEM UNTERDRUCK
UNITE DE DRAINAGE A DEPRESSION REGULEE

(30) Priority: 07.06.1995 US 481237; 05.03.1996 US 12888 P; 07.05.1996 US 17921 P; 05.06.1996 US 19379 P
(43) Date of publication of application: 29.04.1998
(73) Proprietor: SHERWOOD MEDICAL COMPANY, St. Louis, MO 63103-1642 (US)
(72) Inventor: SWISHER, David, R., Maryland Heights, MO 63043 (US); WEILBACHER, Eugene, E., Ellisville, MO 63021 (US); YAM, Jacky, S., St. Louis, MO 63146 (US)
(74) Representative: Rüedi, Regula Béatrice
(86) International application number: PCT/US1996/009792
(87) International publication number: WO 1996/040311

(56) References cited:
- US-A- 3 861 390
- US-A- 4 455 141
- US-A- 4 923 451
- US-A- 4 988 342
- US-A- 5 026 358
- US-A- 5 215 519
- US-A- 5 286 262
- US-A- 5 372 593
- US-A- 5 380 314

## Description

### FIELD OF INVENTION

The present invention relates to a system for draining fluids from the body cavity of a patient, and more specifically to an apparatus for automatically regulating negative pressure in the collection chamber of the system during operation to prevent exposing the patient to dangerously high negative pressure levels. More particularly, this invention relates to an air flow sensitive, buoyant valve that closes when a patient generates the required high air flow rate in the system because of a patient's inspiration, or it shuttles between open and closed positions when a low air flow rate generates excess negative pressure inside the collection chamber.

### BACKGROUND ART

A chest drainage unit is an apparatus for suctioning gases and liquids from the pleural cavity of patients. The pleural cavity lies within the rib cage above the diaphragm and is surrounded by the pleural membrane. The pleural cavity contains both lungs, which in their normal expanded state fill the pleural cavity. Several conditions and diseases such as interventional surgery, trauma, emphysema and various infections can cause a build up of liquid and gases around the lungs in the intrapleural space. When this happens, it causes the lungs to collapse to a volume much less than that of the pleural cavity, thereby severely impairing breathing functions of the patient. The lungs can be re-expanded to their normal state to fill the pleural cavity by draining the liquid and gases from the intrapleural space using a chest drainage unit.

Chest drainage units are also used during autotransfusion for recovering autologous blood from the patient's pleural and mediastinal cavities and transfusing that blood back into the patient. Autotransfusion offers significant advantages over normal transfusion procedures which use homologous blood from other humans. Autologous blood reduces the risk of adverse reactions and transmission of infectious disease while supplying a readily available and safe source of compatible blood to the patient. For these reasons, chest drainage units are being designed to both evacuate fluids from the intrapleural space and autotransfuse shed autologous blood back into the patient.

Various devices have been developed to drain and collect fluids such as blood from the intrapleural space for subsequent autotransfusion. U.S. Patent No. 4,857,042 to Schneider illustrates the prior art development of autotransfusion chest drainage units. The device includes a collection chamber for the collection of fluid from the pleural cavity, a water seal chamber for preventing passage of gas from the atmosphere into the patient's pleural and mediastinal cavities, and a manometer chamber for regulating the degree of vacuum in the system. An inlet port of the collection chamber is connected to the patient's pleural cavity via a thoracotomy tube that deposits shed blood and gases into the collection chamber. The device is also connected to a blood compatible pump at an outlet port of the collection chamber for pumping autologous blood back into the patient. The Schneider device is also provided with a valve mechanism above the water seal chamber to permit the passage of fluids from the water seal chamber in the event of a sudden increase in negative pressure inside the collection chamber, such as when the patient inhales, or blood is withdrawn from the collection chamber with a blood pump or other similar device.

One drawback with the Schneider device is that no provision is made for autotransfusing simultaneously with draining the pleural cavity. Prior art drainage devices generally could not be used to simultaneously collect blood from the pleural and mediastinal cavities and autotransfuse, because there was no provision for automatic regulation of negative pressure during autotransfusion. During continuous autotransfusion, as fluid exits the collection chamber, the remaining fluid volume falls and pressure level concurrently drops within the collection chamber and pleural cavity. It is therefore vital that negative pressure within the collection chamber be maintained within a relatively narrow range to keep bleeding to a minimum and prevent any damage to the intrathoracic tissue which might occur. It is also important to maintain negative pressure within a relatively narrow range in order to prevent water from being transferred out of the water seal chamber and into the collection chamber due to loss of vacuum therein. Permanent loss of water in this manner would render the water seal useless as a one way valve for gases passing out of the collection chamber.

One approach in solving this problem is to provide a collapsible bag whose volume can change as required. U.S. Patent No. 4,443,220 to Hauer et al. discloses such a bag which may be removed from the drainage device when full and placed on a stand to effect reinfusion, however this type of device is incapable of simultaneous drainage and reinfusion. Another method is illustrated by U.S. Patent No. 4,548,413 to Russo wherein a mechanical pressure regulating mechanism in communication with the collection chamber is provided which regulates the sub-atmospheric pressure in the collection chamber independent of the chamber's effective volume. Unfortunately, such regulating mechanisms are costly and often unreliable.

A further problem also arises with the drainage tube which connects the patient to the drainage device. The drainage tube from a patient may itself cause a significant increase in negative pressure in the collection chamber when a nurse attempts to clear or "strip" away any occlusions blocking the tube. "Stripping" is where a nurse or practitioner clears the tube's passageway of blockages that occlude the tube by pinching off a portion of the tube nearest the patient, moving the pinch along the tubing towards the inlet port of the CDU, and then releasing the pinch. This "stripping" action forces any blockages along the tube, but also introduces substantial fluctuations in pressure inside the drainage device due to the sudden release of low pressure in the tube after the pinch is released.

Prior art drainage devices are also designed to permit a patient to draw as much vacuum pressure as is required for both normal and deep inspiration without transferring water from the water seal chamber into the collection chamber. Prior art devices have included push button type valves in communication with the collection chamber for manually venting excess negative pressure inside the drainage device. However, problems exist with manual venting.

One problem associated with manual venting of excess negative pressure is that known venting systems do not give an inherent indication that a high vacuum is present in the collection chamber. Another problem associated with manual venting of excess vacuum is that the nurse must manually push down on the push button valve in order to allow atmospheric air to vent inside the collection chamber. Such a procedure can take upwards of a full minute and requires the nurse to apply constant pressure on the push button valve during release. Further, the nurse must carefully observe and coordinate the level of the water seal with the release of vacuum on the push button valve. Without careful observation and coordination of the manual pressure release during gravity drainage of the collection chamber, the patient's intrathoracic vacuum could be lowered to dangerously low level, such as atmospheric pressure, and cause a serious clinical event to the patient known as a pneumothorax.

One approach to the solution of venting excess negative pressure from the drainage device is illustrated in U.S. Patent No. 5,114,416 to Karwoski et al. The Karwoski et al. device discloses a float valve mechanism interposed between the water seal chamber and the collection chamber that provides for automatic controlled release of excess vacuum beyond that required for patient inspiration. The Karwoski et al float valve allows the patient to draw high vacuum in the collection chamber for breathing while automatically releasing negative pressure when excessive vacuum is maintained for an extended period of time therein. The float valve includes a valve seat forming an aperture and buoyant ball that is adapted to seal against the aperture of the valve seat. During periods when high vacuum exists in the collection chamber due to patient inspiration or deep gasp exercises, water from the water seal chamber rises until it lifts and seats the ball against the valve seat. Once the ball is engaged, the aperture is shaped to permit the water to still pass therethrough but at a substantially reduced rate.

During periods of extended high, excessive vacuum in the collection chamber the water column becomes depleted as enough water is forced up one arm of the water seal chamber and passes through the aperture. Once through the aperture, the water flows into an overflow chamber and the height of the water column below becomes insufficient to maintain the ball valve in the seated position due to leakage of ambient air into the collection chamber following the final influx of water therethrough, thereby effecting automatic release of the valve. However, the Karwoski et al. device suffers from several drawbacks.

The Karwoski et al. floating valve is activated by hydraulic pressure of the water column from the water seal lifting and engaging the valve ball into the sealed position. Unfortunately, the design of the Karwoski et al. valve permits leakage of fluid when engaged in the sealed position. This leakage eventually allows air to flow into the collection chamber during extended deep gasp exercises and effectively lowers the negative pressure therein over time, thus making it difficult for the patient to maintain a constant negative pressure inside the collection chamber during the deep gasp exercise. During convalescence, it is preferable to maintain a constant negative pressure inside the pleural cavity in order to allow the patient to effectively exercise the diaphragm muscles under the cavity. Without a sufficient negative pressure, the diaphragm muscles are unable to exert enough muscular action against the pleural cavity due to the underinflated condition of the lungs.

The Karwoski et al. device also suffers from other drawbacks. Although this device provides automatic negative pressure relief during low flow situations like continuous autotransfusion, the valve is not designed to be air-flow sensitive and is incapable of maintaining excessive vacuum when the patient is performing extended deep gasp exercises. Often during convalescence of the pleural cavity, patients are required to perform deep gasp exercises in order to strengthen the diaphragm muscles beneath the lungs. In performing these exercises a sudden and high excessive vacuum is generated in the collection chamber when the patient takes a sudden and deep inspiration. When using the Karwoski et al. device, the patient is incapable of maintaining the required high vacuum inside the pleural cavity during an extended deep gasp exercise because the float valve does not remain closed. Instead, the valve leaks water after the water column from the water seal chamber engages and seals the float valve, and air continues to leak past the valve after the water is depleted. Thus, the Karwoski's float valve operates only when the water column is drawn upward and engages the valve into its sealed position, and the valve will always leak air even after the water column under it is depleted.

Furthermore, in US 5,286,262 to Karowski et al., a chest drain device is disclosed that has the same basic structure and the same float valve as shown in US 5,114,416 to Karowski et al.

Accordingly, it is the principle object of the present invention to provide a fluid recovery system having an improved flow-sensitive, buoyant valve that provides for automatic and/or complete closure when a minimum amount of air flow is applied thereto.

Another important object of the present invention is to provide an air-flow sensitive valve that maintains substantially the same negative pressure inside the collection chamber of the apparatus as is being maintained in the patient's pleural cavity while the patient is performing deep gasp exercises.

It is also an object of the present invention to provide an improved float valve that does not leak during operation.

Another further object of the present invention is to provide a buoyant valve that can operate to relieve excess negative pressure when there is a slow rate of increase in negative pressure within the apparatus.

It is a further object of the present invention to provide a buoyant valve that will shuttle between open and closed positions in such a manner that excess negative pressure is automatically relieved when there is a slow rate of increase in negative pressure.

Another object of the present invention is to provide a valve that combines the dual functions of relieving excess negative pressure during low flow rate conditions while maintaining equilibration of the collection chamber and pleural cavity pressures during high air flow rate conditions.

### DISCLOSURE OF INVENTION

The invention is as defined in the appended set of claims.

The present invention provides for a disposable unitary structure for sterile collection of fluids from the pleural cavity of a patient, and if desired, for simultaneously reinfusing such fluids back to the circulatory system of the patient. The apparatus comprises a rigid collection chamber for receiving fluids drained from the pleural cavity, a U-shaped water seal chamber for preventing passage of atmospheric air into the collection chamber, and a manometer chamber for regulating the amount of vacuum inside the collection chamber. In the preferred embodiment, the collection chamber has three ports: the first port is adapted for connection to a thoracotomy tube for draining fluids from the pleural and mediastinal cavities into the collection chamber; the second port communicates with the water seal chamber; and the third port is adapted for connection with an infusion pump and separate collection vessel for delivery and reinfusion of collected fluids back into the circulatory system of the patient.

The apparatus is configured to operably maintain a selected negative pressure range in the collection chamber during the outflow of collected fluid, and to permit reinfusion of collected blood from the collection chamber simultaneously with drainage from the pleural or other body cavity into the same chamber. Alternatively, the apparatus may be used as a means of facilitating patient convalescence. This is accomplished by maintaining a substantially constant negative pressure condition inside the collection chamber when the patient exercises the diaphragm muscles through deep extended gasp exercises during convalescence.

The present invention also includes an improved air-flow sensitive float valve interposed between the collection chamber and one arm of the water seal chamber for allowing the patient to draw as much vacuum pressure as is required during deep inspiration. The float valve also functions to provide for an automatic controlled release of excess negative pressure in the collection chamber during either continuous autotransfusion or when there is a low air flow rate condition present. By tailoring a specific amount of water to a given size chamber, the water column inside the water seal chamber is allowed to'momentarily engage the floating mechanism before the water seal chamber is emptied of water and a column of air is subsequently forced through the water column towards the momentarily sealed valve. Once the air column reaches the valve, the water supporting the valve is displaced and the valve is released until the air column is replaced by another rising water column. This displacement of air through the water column causes the valve to "shuttle" between open and closed states as the alternating rising water and air pocket engages the valve so that sub-atmospheric pressure in the collection chamber is maintained and excess negative pressure is relieved.

In another mode, the valve has an air flow-sensitive feature that permits activation of the valve during high air flow conditions without the valve having to be engaged by the column of water required in the Karwoski et al system. When a patient takes a sudden and deep inspiration, a pocket of air normally precedes the rising water column up the one arm of the water seal chamber towards the collection chamber. The air-sensitive valve of the present invention is designed with a valve body that is sensitive to air flow and is designed to lift the valve towards a sealing engagement with the sealing member when the air column reaches the valve chamber.

This valve design automatically restores the patient back to minimum lower vacuum threshold and provides a simple system of automatic release of excess negative pressure that does not leak fluid nor require any monitoring by an operator when the chest drainage unit is operating under continuous autotransfusion. In addition, the valve design permits more effective convalescence of the patient during chest drainage due to the air-flow sensitive valve feature that closes during high air-flow conditions when a rising air column caused by the patient performing deep gasp exercises engages the valve.

### BRIEF DESCRIPTION OF DRAWINGS

FIG. 1 is a simplified block diagram showing the basic operation of a prior art autotransfusion system;
FIG. 2 is a partial cross section showing an exploded view of an autotransfusion chest drainage unit in accordance with the present invention;
FIG. 3 is a partial cross section of the water seal chamber showing the operation of the valve in accordance with the present invention;
FIG. 4 is a perspective of the valve in accordance with the present invention.
FIG. 5 is a partial cross section showing the sealing engagement of the valve to the valve seat in accordance with present invention;
FIG. 6 is a cross section of the water seal chamber showing the shuttling action of the valve in the open position in accordance with the present invention;
FIG. 7 is a cross section of the water seal chamber showing the shuttling action of the valve while in the closed position in accordance with the present invention.
FIG. 8 is a perspective illustrating the method of using the spike valve according to the present invention.
FIG. 9 is a cross section of the Y-site connector according to the present invention.
FIG. 10 is a perspective of the access valve according to the present invention.
FIG. 11 is a cross section of the access valve according to the present invention.

### MODE(S) FOR CARRYING OUT THE INVENTION

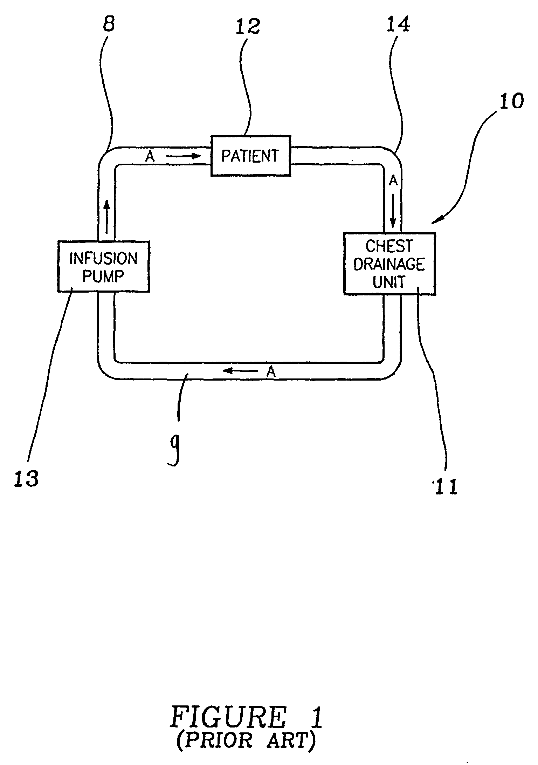

A prior art autotransfusion system in accordance with the present invention is shown in FIG. 1. The basic configuration of an autotransfusion system **10** comprises a chest drainage unit (CDU) **11** for sterile collection and if desired, transfer of shed fluids from a patient **12**, a blood compatible infusion pump **13** connected to the CDU **11** for reinfusing shed blood back to the patient **12**, and infusion tubing **14** for use as a conduit to transfer the blood between the autotransfusion system **10** and the patient **12**. Liquid flow A denotes the direction of the fluid flow within system 10.

The basic operation of the autotransfusion system **10** is disclosed in U.S. Patent No. 4,798,578 to Ranford.

In short summary, the autotransfusion system 10 operates by using CDU **11** for the sterile collection of blood and fluids drawn from patient **12,** and simultaneous reinfuses the fluids back to the circulatory system of patient **12**.

The general process of transfusing a patient's blood begins by drawing fluids from the patient's pleural cavity using a suction source (not shown) located at CDU **11** to create a positive liquid flow A through the autotransfusion system **10**. The suction forces shed body fluids from patient **12** through infusion tubing **14** and into the collection chamber (not shown) of CDU **11.** As fluid enters the collection chamber **15** at collection port **28**, it is run through a gross filter (not shown) which traps macroscopic debris such as blood clots, bone fragments and the like that become entrained in blood or other body fluids. Once the fluid is filtered, it is temporarily stored in the collection chamber 15 where it is again filtered using a microaggregate filter (not shown) as the fluid exits the CDU **11.** In alternative embodiments, the egress of blood from the collection chamber **15** can be from any suitable site along the surface of the collection chamber where the infusion tubing **9** may be attached. Once the blood is filtered through the filter, it goes to infusion pump **13** where the blood is reinfused back to patient **12** through infusion tubing (9, 8), thereby finishing one complete autotransfusion cycle. Infusion tubing **14** may be made of any suitable flexible plastic material, for example polyurethane or PVC, for use in transmitting fluids and gas throughout system **10**.

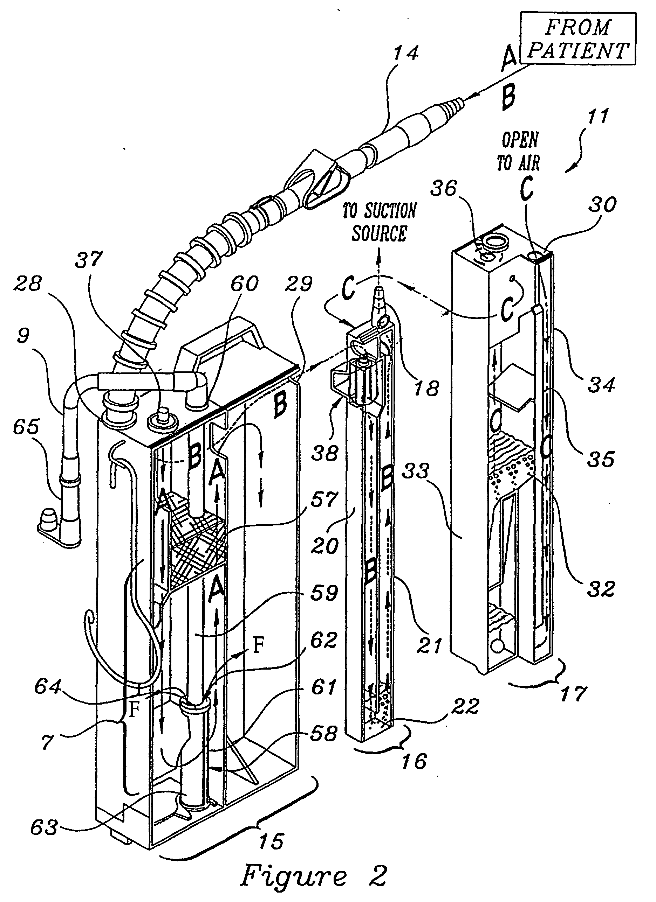

Referring to FIG. 2, the operative features of CDU **11** in accordance with the present invention will be discussed in greater detail. An autotransfusion CDU **11** consists of a standard three chamber unit found in the prior art comprising a blood collection chamber **15**, a water seal chamber **16** and suction control chamber **17**. The blood collection chamber **15** is designed to receive fluid drained from the patient's pleural and mediastinal space, but it may also function as a filtration site to filter blood and other fluids of unwanted debris. In an alternative embodiment, CDU **11** may be configured to have an additional second collection chamber (not shown) separate from the CDU **11** body which acts as the filtration site while the collection chamber **15** inside CDU **11** acts as an overflow chamber for the second detached chamber, as disclosed in the aforementioned Ranford patent.

The other two chambers, the water seal chamber **16** and suction control chamber **17**, serve to control and regulate the liquid flow A inside the collection chamber **15** as well as the pressure inside CDU **11**. The suction control chamber **17** provides regulation of negative pressure during operation. Negative pressure within the CDU **11** is controlled by the height of water **32** in the suction control chamber **17** which insures a continuous suction of the pleural cavity and also alleviates concerns over possible tissue invagination in the thoracic catheter during high levels of negative pressure within the cavity.

As further shown in FIG. 2, the suction control chamber **17** consists of a U-shaped chamber having first and second arms **33**, **34** respectively. A column of water **32** fills the bottom portion of the suction control chamber **17** and extends upward through both arms **33**, **34**. First arm **33** is in communication with both a second arm **21** of the water seal chamber **16** and a suction source (not shown) while the second arm **34** is open to atmospheric air which maintains an area of atmospheric pressure inside the arm **34** above the water line **35**. Air flow C denotes the air flow throughout the suction control chamber **17**. Air flow **C** shows atmospheric air being pulled into the second arm **34** through open port **30** and into first arm **33** where it exits arm **33** through the suction port **18** towards the suction source. The height of the column of water **32** interposed between the first arm **33** exposed to vacuum source pressure and the second arm **34** which is at atmospheric pressure determines the level of negative pressure inside the collection chamber **15** and water seal chamber **16**. For example, 20 cm of water **32** at the column translates to a negative pressure of -20 cm inside the collection chamber **15**. U.S. Patent No. 4,439,190 to Protzmann et al. gives a more detailed description of a typical suction control chamber of a CDU **11** and its operation.

The water seal chamber **16** prevents reflux of air and fluid back to the patient by preventing the reentry of air and fluid into the collection chamber **15** using an air sensitive, buoyant valve **38** in combination with a water seal **22.** As shown in FIG. 2, the air flow **B** is created by applying the source of suction to suction port **18** located at the top of the water seal chamber **16.** The applied suction creates an air flow **B** that forces fluid from the patient's body (not shown) through the infusion tubing **14** and into the top of the collection chamber **15** at port **28** where air flow **B** passes through opening **29** and into the water seal chamber **16**. Once inside the water seal chamber **16**, air flow B travels down a first arm **20** and through the water seal **22** located at the bottom portion of chamber **16**. After passing through the water seal **22**, air flow B travels up second arm **2**1 where it exits at suction port **18**. Thus, air flow **B** creates a positive flow path that forces gases out of collection chamber **15** and through water seal **22** where these gases are removed from CDU **11** through suction port **18.**

The first arm **20** of the water seal chamber **16** is in fluid flow communication with second arm **21** through water seal **22** at one end and the collection chamber **15** at the other end, while the second arm **21** is in fluid flow communication with the first arm **20** at one end and the suction control chamber **17** at its other end respectively. Water seal **22** functions as a protective one way valve that allows air to escape from the collection chamber **15,** while preventing contaminated atmospheric air from reentering the pleural cavity of the patient. By interposing a water seal **22** at the bottom portions of both first and second arms **20**, **21**, fluid is prevented from passing back through the water seal **22** due to the difference in pressure maintained between the two arms **20**, **21**, thus preventing a reflux action. The importance in preventing reflux is that under certain respiratory conditions, a sudden increase in pressure within the pleural cavity can appear. For example, an air leak in the pleural cavity can interfere with the normal respiratory function of the patient's lungs. Finally, the bubbling action shown in the water seal **22** represents evacuated air from the collection chamber **15** that has passed through seal **22** into second arm **21**.

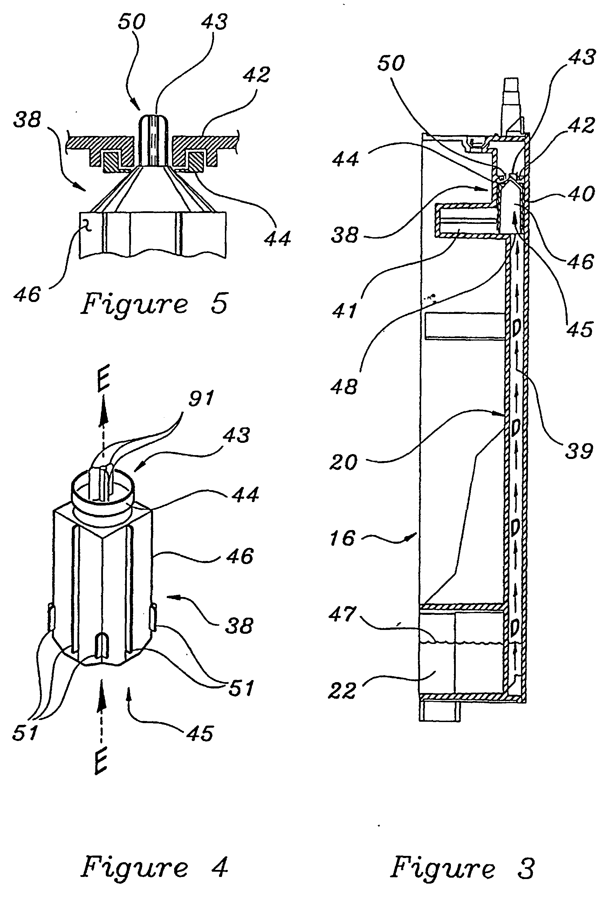

In addition to water seal **22**, which prevents any reflux of gases back into the collection chamber **15**, an air flow sensitive, buoyant valve **38** is provided at the top portion of water seal chamber **16** as a further safeguard. Referring to FIG. 3, valve **38** is positioned at the top portion of column **39** of water seal chamber **16** which includes a valve chamber **40** and accumulation chamber **41**. The top portion of valve chamber **40** defines a valve seat **42** which forms an aperture **50** therethrough and is shaped for air-tight engagement with valve **38** when valve **38** is seated therein. CDU **11** is also designed so that air can escape through an automatic positive pressure relief valve **36** when an overpressure condition occurs inside the patient's pleural cavity, e.g. when the patient coughs. The automatic positive relief valve **36** is positioned on the top portion of CDU **11** and is in fluid flow communication with the suction control chamber **17.** Similarly, the collection chamber **15** has a negative pressure relief valve **37** which manually vents excess negative pressure from the patient's pleural cavity when actuated by a nurse.

In addition to air flow **B**, there exists an air flow **C** which is ambient or atmospheric air that enters the CDU 11 through open port **3**0 and forced through the suction control chamber **17** and into the top portion of the water seal chamber **16** where air flow **C** exits through suction port **18**.

During an overpressure condition in collection chamber 15, accumulation chamber **41** functions to hold excess water that rises up the column **39** and also serves as a platform for holding valve **38** in place when the valve **3**8 is disengaged from valve seat **42**.

With reference to FIG. 4, a more detailed description of valve **38** will be discussed. Valve **38** has a rectangular hollow-shaped configuration that comprises a valve body **46** and a separate grommet **44** adapted to fit over the guide **43**. In the preferred embodiment, the valve body **46** has a top portion that forms a guide **43** and four side walls that form an interior chamber **45** therein. Interior chamber **45** is designed to be air flow sensitive and allows the valve **38** to be lifted upward into sealing engagement with the valve seat **42** when either a sudden reflux of air in the direction of air flow **E** occurs or a slow rise in the water seal **22** lifts and engages valve **38** which causes an excessive negative pressure condition in the collection chamber **15**. Although the valve **38** has a rectangular shape, any suitable shape design having the aforementioned aerodynamic properties is felt to fall within the scope of the present invention.

With reference to FIG. 5, valve **38** is provided with grommet **44** for sealing the valve **38** against the valve seat **42** in fluid-tight engagement therein. Preferably, the grommet **44** has a ring shape design that fits over the guide **43** and is interposed between the valve body **46** and the valve seat **42** in such a manner that the grommet **44** effectively seals the aperture **50** from fluid flow communication therethrough when the valve **38** is engaged therein. The grommet **44** may be made of any suitable flexible plastic material, for example polyurethane or silicone, that effectively seals the valve **38** against the valve seat **42** from fluid flow communication when engaged.

Referring back to FIG. 4, guide **43** is provided with a plurality of fins **91** that facilitate guide **43** through the aperture **50** when valve **38** is being seated against valve seat **42.** To further enhance the aerodynamic design of valve **38,** a plurality of flanges **51** are also provided at each bottom corner and side wall of valve **38** for influencing the air flow through valve chamber **40** and around valve **38.**

With reference to FIG. 3, a detailed description of the operation of valve **38** will be discussed. Valve **38** operates in two modes: (1) during low air flow rate conditions in which excessive negative pressure increases at a relatively gradual rate, such as during autotransfusion, valve **38** prevents entry of rising water from the water seal **22** from entering the collection chamber **15**; and (2) during high air flow conditions in which excessive negative pressure builds up at nearly an instantaneous rate, such as when a patient takes a rapid deep inspiration or "gasp", valve **38** prevents ambient or atmospheric air from the water seal chamber 16 from entering the collection chamber **15**.

In the first mode, valve **38** operates to prevent the ingress of water from the water seal **22** into the collection chamber **15**. During autotransfusion an excess negative pressure slowly builds up in the patient's pleural cavity which gradually forces water within water seal **22** to rise up column **39** until it engages valve **38**. This forced rising of water is due to the pressure differential between the collection chamber **15** and the second arm **21** of the water seal chamber **16** and is generated by operation of infusion pump **13**. First water level **47** denotes the original water level in water seal **22** before autotransfusion process draws water up column **39**, while flow path **D** denotes the direction of the rising water. When autotransfusion begins, the low air flow rate condition will cause the water level to slowly rise up column **39** from its first water level **47** until it reaches a second water level **48** and engages valve **38**. Once valve **38** is engaged, the further rising water will begin filling both the accumulation chamber **41** and interior chamber **45** and valve **38** will begin to rise up the valve chamber **40** until valve **38** is fully seated against the valve seat **42**, thereby preventing fluid flow communication therethrough.

After valve **38** has seated itself against valve seat **42** in response to the low air flow rate condition caused by autotransfusion, the water seal chamber **16** of the present invention is specifically configured to permit "shuttling" of valve **38** during the remainder of the autotransfusion procedure. In this manner, further excess negative pressure is not allowed to build up in collection chamber **15**. Shuttling as described herein is the up and down motion of valve **38** as it shuttles between closed and open positions in response to this slow increase in excess negative pressure generated by infusion pump **13**. Although shuttling is described as an up and down motion of valve **38**, any back and forth action that closes and opens valve **38** is felt to fall within the scope of the present invention. Shuttling prevents the accumulation of excess negative pressure in the collection chamber **15** after valve **38** has been initially closed by the rise of the water in column **39**. By allowing small amounts of ambient air to pass into chamber **15** when the valve **38** has shuttled to an open position, the chamber **15** is automatically relieved of further excess negative pressure therein.

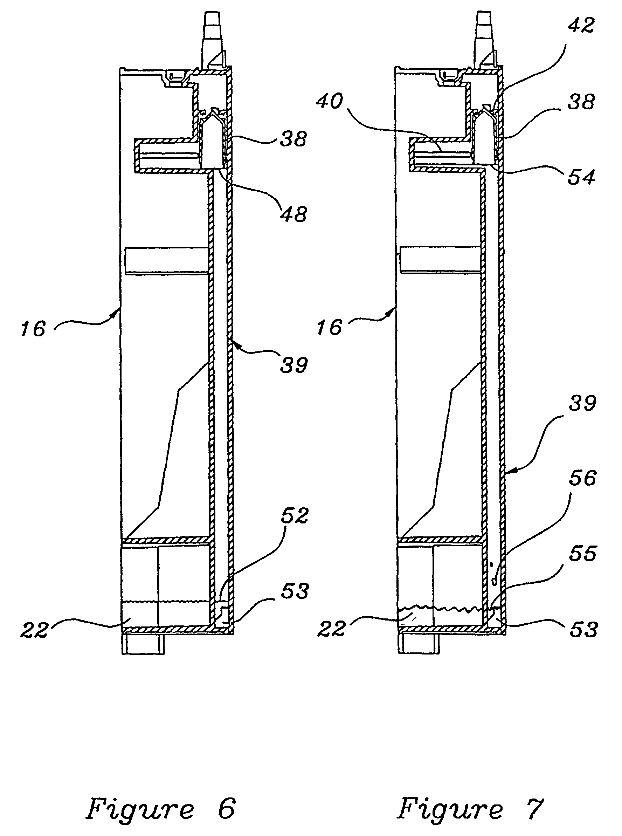

Referring now to FIGS. 6 and 7, a detailed description of the shuttling operation of valve **38** within the water seal chamber **16** will be explained. FIG. 6 shows valve **38** in the open position during low air flow conditions wherein water within the water seal **22** is forced up through column **39** due to the aforementioned slow increase in excess negative pressure within collection chamber **15.** Water seal chamber **16** is configured in such a manner that a predetermined volume of water in water seal **22** is sufficient to completely fill up column **39** and engage valve **38** to valve seat **42** in sealing engagement therein, but not sufficient to maintain continuous engagement of valve **38**. As seen in FIG. 6, the water has reached water level **48** prior to engagement of valve **38** to valve seat **42** while water in the water seal **22** has been correspondingly lowered to water level **52** just above seal opening **53.** With reference to FIG. 7, if the slow increase in excess negative pressure continues the water will rise up column **39** until it reaches a water level **54.** Once water level **54** has been reached valve **38** is placed in sealing engagement with valve seat **42,** thereby closing valve **38** to fluid flow communication therethrough. When valve 38 is closed, water within water seal **22** will be further lowered to a water level **55** which places level **55** to a height equal to seal opening **53.** By design, the height of water level **55** in relation to seal opening **53** causes air pockets or bubbles of air **56** to flow from the second arm **21** of water seal chamber **16** through seal opening **53** and up column **39**. The rising air pocket **56** eventually displaces the water that closes off valve **38** because of the lack of hydraulic pressure support, thereby momentarily releasing it from its closed position.

Once valve **38** is released, rising water quickly replaces the air pocket **56** and valve **38** is again closed to fluid flow communication therethrough. This shuttling process alternates between the water that closes valve **38** and the air pocket **56** that opens it as long as the autotransfusion procedure or other of the aforementioned low air flow rate conditions persists. The shuttling action of valve **38** automatically maintains the desired negative pressure level inside collection chamber **15** until the procedure is terminated. In short, the present invention specifically tailors the volume of water in the water seal chamber **16** to a predetermined amount of water in the water seal **22**, thereby generating air pockets **56** that alternate with the rising water to shuttle valve **38** between open and closed positions.

As mentioned briefly before, the slow rate of increase in the negative pressure inside collection chamber **15** is what causes the water to initially rise within water seal chamber **16**. This slow excess negative pressure buildup can be generated by the operation of infusion pump **13** during autotransfusion, the drawing of a sample from a Y-site connector placed in fluid flow communication with infusion tubing **9** leading from collection chamber **15**, or by utilizing a separate blood bag during autotransfusion as disclosed in U.S. Patent No. 4,033,345 to Sorenson et al.. Therefore, valve **38** is adapted to work in one of several operating environments where there is a slow rate of increase in the excess negative pressure inside the collection chamber **15** that causes a low air flow condition to arise which forces water in water seal **22** to slowly rise and migrate up column **39**.

In the second mode of operation, valve **38** activates to close when an excessive negative pressure build up occurs nearly instantaneously such as in a high air flow rate generated by the patient taking a sudden and extended deep inspiration or "gasp". When a deep inspiration is performed, the patient has the potential of exerting a negative pressure of approximately -60 cm H₂O inside collection chamber **15**. This generates a high air flow rate condition moving in a direction of air flow D which results in a high pressure differential between the chamber **15** and the rest of CDU **11**. In response to this condition, valve **38** rises to its closed position and remains closed until the patient releases the deep inspiration. This closed action occurs well before the water in column **39** can rise sufficiently to lift valve **38.**

Valve **38** is adapted to close and open in response to any sudden inspiration by the patient. In the preferred embodiment, valve body **46** has a hollow-shaped configuration that is aerodynamically designed to engage valve seat **42** when the aforementioned high air flow condition is applied to the interior chamber **45**. However, any suitable valve configuration that is adapted to close valve **38** when a high air flow condition is applied thereto in the direction of air flow **D** is felt to fall within the scope of the present invention.

A detailed operation of valve **38** during high air flow conditions will now be discussed. During convalescence when the patient takes an extended deep inspiration or "gasp" exercise, a high air flow condition within column **39** will exist in the direction of air flow **D** which enters valve chamber **40** and lifts valve body **46** into air-tight sealing engagement with valve seat **42**. Once engaged, valve **38** will remain closed until the patient stops the deep inspiration which in turn releases and opens valve **38** from sealing engagement therein. This sealing engagement of valve **38** during high air flow conditions serves to maintain an equilibration in negative pressure between the collection chamber **15** and the patient's pleural cavity while aiding the patient to convalescence the pleural cavity.

The method of collecting and reinfusing fluids back to a patient using valve **38** as a safeguard will now be discussed. The steps of collecting and reinfusing fluids to a patient first requires the practitioner to fill the water seal chamber 16 with approximately 40 - 42 cc of liquid. In the preferred embodiment, a pre-attached burette (not shown) having a volume in the range of 40 - 42 cc is located with CDU **11** so that the practitioner need only completely fill the burette and transfer the predetermined amount of liquid into the water seal chamber **16.** In alternative embodiments, any suitable device that allows the practitioner to fill the correct amount of fluid and deposit or inject the same into water seal chamber **16** can be used. After filling water seal chamber **16,** suction control chamber **17** is filled with liquid to a level adequate to maintain the proper negative pressure level inside CDU **11** required by the practitioner in a manner well known in the art.

Once the proper amount of negative pressure has been applied to inside of CDU **11** and the correct amount of liquid has been deposited in water seal chamber **16,** infusion tubing **14** is attached to collection port **28** and a thorácic catheter (not shown), which has been inserted into the patient's pleural and mediastinal cavities, is attached to tubing **14.** At that time, blood is collected in the collection chamber **15** by means of either gravity or suction drainage over a period of time. After a predetermined amount of blood has been collected, the practitioner may then order the autotransfusion procedure to commence. In order to establish the autotransfusion procedure, further infusion tubing **9** is used to place the outlet port **60** of collection chamber **15** in fluid flow communication with the inlet portion of infusion pump **13** while other tubing **8** is used to attach the outlet portion of pump **13** in fluid flow communication with the circulatory system of patient **12**, thereby completing the assembly of system **10**.

After completing assembly of system **10**, a source of suction is applied to the suction port **18** and the infusion pump **13** is activated. Once the infusion pump **13** is activated, there will be a slow increase in negative pressure inside collection chamber **15** and a slow air flow rate condition will exist in the water seal chamber **16**. Water from water seal **22** will slowly rise up column **39** until it engages valve **38**, thereby starting the shuttling action of valve **38** between closed and open positions until such time as the infusion pump **13** is shut off.

Referring back to FIG. 2, a detailed description of the filtration system **7** in accordance with the present invention will be discussed. Filtration system **7** provides dual filtration of shed blood at both the inlet and outlet portions of collection chamber **15** and comprises gross filter **57,** filter assembly **58** and drop tube **59** that places assembly **58** in fluid flow communication with outlet port **60.** Gross filter **57** is interposed between the inlet port **28** and filter assembly **58** and serves to remove blood clots, bone, fat and bowel contents that become entrained in the blood as the blood enters from inlet port **28**. Preferably, gross filter **57** is a large area gross filter, such as a fabric or an open-pore sponge filter, that is suitable for removing blood clots and gross particulates from blood.

Once the blood begins to fill the bottom portion of collection chamber **15,** it is refiltered by filter assembly **58** as it exits chamber **15** for reinfusion back to the patient. Filter assembly **58** includes a fine filter **61** for removal of formed clots and a valve cap **62** that is attached to the top portion of filter **61.** Valve cap **62** functions both as a retaining member for securing drop tube **59** as well as a one way valve for clearing out later forming blood clots that may clog either drop tube **59** or infusion tubing **9** leading from the collection chamber **15** once the blood has been filtered through fine filter **61.**

Referring to FIG 2, a more detailed description of filter assembly **58** will be discussed. As mentioned briefly above, filter assembly **58** comprises a fine filter 61 and valve cap **62.** Fine filter **61** functions as a secondary filtration site for removing blood clots that form after filtration through gross filter **57** as the blood sits and collects at the bottom portion of collection chamber **15** prior to reinfusion. A micron polyester screen **63** is provided around substantially the entire surface area of fine filter **61** and provides a small pore size media for removing blood clots that form inside the collection chamber **15.** Preferably, fine filter **61** is a model 991-66 high flow blood filter with a 210 micron screen manufactured by CUTTER BIOLOGICAL of Berkeley, California, however any suitable blood filter with a micron screen ranging from 40 to 210 microns is felt to fall with the scope of the present invention.

During manufacturing, drop tube **59** is attached to outlet port **60** at its distal end while the proximal end of tube **59** is aligned and secured through a cylindrical bore **64.** The proximal end of drop tube **59** forms a collection opening (not shown) that is confined inside filter assembly **58** and is spaced approximately 0,9325 cm (3/8 inch) off the collection chamber floor (not shown). Collection opening provides an inlet for receiving fluid flow therein during the filtration process wherein collected blood is forced through the collection opening and into drop tube **59** for reinfusion to patient **12.** As mentioned above, the collection opening is preferably spaced 0,9525 cm (3/8 inch) off the collection chamber floor, however any suitable spacing distance which allows for sufficient suctioning of collected fluid during filtration is felt to fall within the scope of the present invention.

The functional aspects of valve cap **62** will now be discussed. valve cap **62** serves as a retaining member for securing the proximal end of drop tube **59** to filter assembly **58** and also functions as a one way valve. The one-way valve action of valve cap **62** permits the reflux of trapped air or blood that has developed clots after filtration by fine filter **6**1 back into the collection chamber **15**. This reflux action clears out the blood clots and trapped air from the fluid pathway leading from fine filter **61**. According to the present invention, the fluid pathway comprises the conduit through drop tube **59** inside collection chamber **15** and the portion of infusion tubing **9** interposed between the outlet port **60** of CDU **11** and the patient **12.**

To facilitate the reflux action, valve cap **62** includes a filter disc **64** that is similar to the micron polyester screen **63** disclosed above. Filter disc **64** has a donut shaped configuration and is bonded to the partition member (not shown) at a plurality of points near the inner circumference of disc **64.** Whenever clots form or air becomes trapped inside drop tube **59** or tubing **9** after passing through fine filter **61,** filter disc **64** functions as a one way valve. The flexibility of the polyester screen of filter disc **64** permits the outer circumference of disc **64** to lift up and allow clotted blood or trapped air to be flushed back into collection chamber **15** whenever a reflux action is initiated by the practitioner. This reflux action is initiated by the practitioner who places a syringe or other type of flushing means (not shown) in fluid flow communication with infusion tubing **9** attached to outlet port **60.**

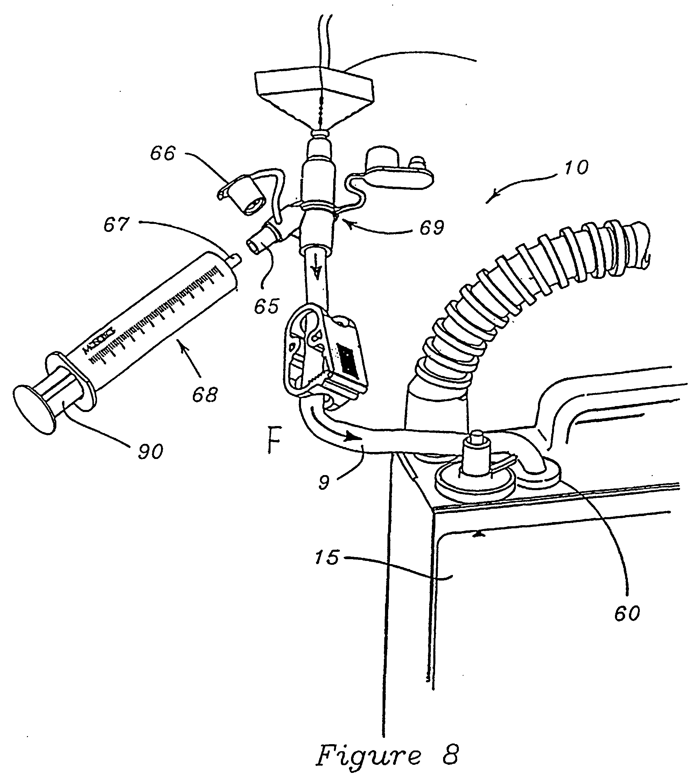

Referring now to FIG. 8, the preferred method of clearing the fluid pathway of blood clots and trapped air inside autotransfusion system **10** will now be discussed in greater detail. The method of flushing out large blood clots and trapped air from the fluid pathway is initiated by the practitioner turning off infusion pump **13** so that fluid flow in the direction of patient **12** is interrupted. After infusion pump **13** is turned off, a slide clamp is used to pinch off infusion tubing **9** downstream of microaggregate filter from fluid flow communication therethrough. The practitioner then uncaps dust cap/plug combination **66** and inserts a luer tip **67** of flushing means **68** inside a spike valve **65** that is in fluid flow communication with the fluid pathway through Y-Site connector **69.** In the preferred embodiment, spike valve **65** is a BESPAK valve manufactured by Bespak of Cary, North Carolina.

Preferably, the flushing means **68** is a syringe that includes a plunger **90** and luer tip **67** and contains a solution of sterile saline, however any suitable type of flushing means that forces a fluid medium through the fluid pathway in a clearing action is felt to fall within the scope of the present invention.

Once the luer tip **67** is inserted and engaged to spike valve **65** and plunger **90** is in the full pull back position, the practitioner pushes plunger **90** forward until all of the saline is released into the fluid pathway. The release of the saline forces any blood clots or trapped air back through the fluid pathway in the direction denoted by fluid flow **F**.

Referring back to FIG. 2, a more detailed description of the aforementioned flushing action in relation to valve cap **62** will be discussed in greater detail. After the practitioner has initiated the flushing action in the direction of fluid flow **F**, blood clots and trapped air contaminating the blood are forced back through the fluid pathway and into collection chamber **15** through outlet port **60.** Once through outlet port **60**, the contaminated blood travels through drop tube **59** where it exits at the collection opening and into an interior chamber of filter assembly **58.** This flushing action produces a positive pressure within filter assembly **58** which forces open the outer circumference of filter disc **64** in a flapping action that forces out the contaminated blood back into collection chamber **15** while also producing a limited fluid flow through the filter media. After the flushing action has subsided, the outer circumference is restored to its original resting position, whereby the polyester screen of filter disc **64** will function as a filtration site, along with fine filter **61**, to refilter the contaminated blood.

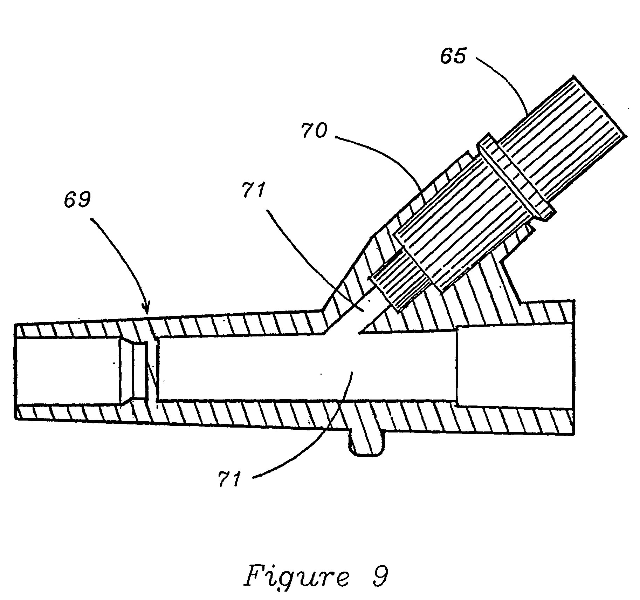

Referring now to FIG. 9, a cross section of Y-site connector **69** is shown with access port **70** in fluid flow communication with the interior chamber **71** of connector **69**. The access port **70** permits the practitioner access for the purpose of priming, sampling, aspirating or injecting medication into system **10**. Two-way valve **65** is partially disposed within access port **70** for accessing interior chamber **71**. The valve **65**, shown in perspective in FIG. 8, is preferably a BESPAK valve manufactured by Bespak of Cary, North Carolina, which is a luer activated valve that is automatically closed to fluid flow communication whenever the luer tip of the syringe is removed from the distal opening of valve **65**. Normally used as a valve for inflating the distal end of catheters inside patients in order to prevent removal of the catheter, the applicants are utilizing the BESPAK valve **65** for the novel purpose of permitting two-way access into an autotransfusion system **10.**

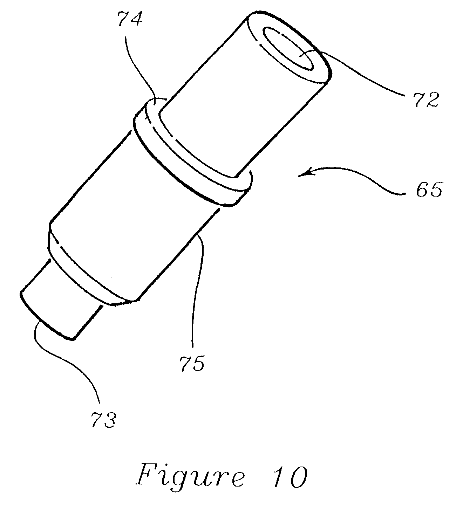

As shown in FIG. 10, valve **6**5 has a tubular shape that comprises an annular distal opening **72**, a proximal opening **73** and a flange **74**. The flange **74** is positioned around the middle portion **75** of valve **65** and serves to hold the valve **65** in place inside the distal end of spike port **70** by having the flange **74** abut against the lip of the spike port **70**.

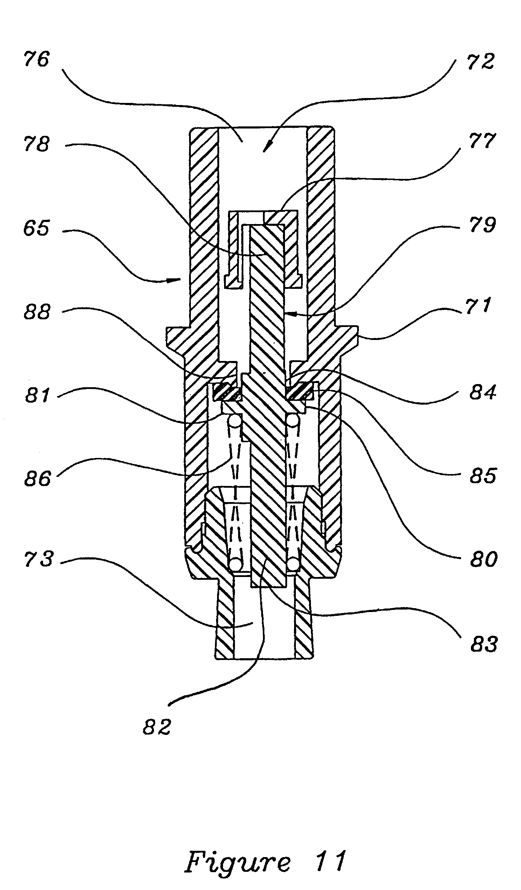

Referring now to FIG. 11, the basic operation of BESPAK valve **65** will be discussed. Valve **65** is a spring loaded valve that is normally closed to fluid flow communication. Distal opening **72** opens into a first interior chamber **76** where an activation piece **77** is disposed therein and attached to the distal end **78** of push rod **79**. Rod **79** comprises distal end **78** that is disposed within first interior chamber **76**, a middle portion **80** that includes an annular flange **81** and a proximal end **82** which are all disposed within a second interior chamber **83**. The first interior chamber **76** and second interior chamber **83** are separated by aperture **84** which permits rod **79** to pass therethrough. Middle portion **80** includes a rubber seal **85** on a first side of portion **80** that seals the aperture **84** from fluid flow communication therethrough between first interior chamber **76** and second interior chamber **83**. The distal end of spring means **86** is attached to a second side of middle portion **80** while the proximal end of spring means **86** is attached to the walls forming the proximal opening **73**. Spring means **86** creates a continual forward bias towards distal opening **72**, so that rubber seal **85** closes off fluid flow communication between first and second interior chambers **76** and **83** unless push rod **79** is properly activated.

Activation of push rod **79** is accomplished by the practitioner inserting the luer tip of a syringe (not shown) into the distal opening **72** of valve **65** until the luer tip contacts the activation piece **77.** As the practitioner pushes downward onto the activation piece **77,** the push rod **79** along with rubber seal **85** is moved towards the proximal opening **73,** thereby opening aperture **84** and permitting fluid flow communication therethrough between the first interior chamber **76** and first interior chamber **83.** Once activation piece **77** of rod **79** abuts interior flange **88,** the forward motion of the luer tip is stopped and the syringe is fully engaged with valve **65** and ready for use.

Although particular embodiments of the inventions have been shown, it is not intended that the inventions be limited thereby, instead, the scope of the present invention is intended to be limited only by the appended claims.

## Claims

1. A chest drainage unit (11) for the collection of fluids, defining first, second and third intercommunicating chambers (17,16,15), said chest drainage unit (11) being adapted to be interposed between, and in fluid flow communication with, an infusion pump (13) and a patient (12), said third chamber (15) being a rigid collection chamber, said second chamber (16) being a U-shaped water seal chamber, and said first chamber (17) being a manometer or suction control chamber,
said chest drainage unit (11) having first, second and third openings (30, 18, 28) for communication with atmosphere, a vacuum source, and a fluid collection tube (14), respectively,
a first water column (32) being included in said first chamber (17), said second chamber (16) comprising a first arm (20) and a second arm (21), a water seal (22), and an air flow sensitive buoyant valve (38),
said first arm (20) being in fluid flow communication with said second arm (21) through said water seal (22) at one end and with said third chamber (15) at the other end,
said second arm (21) being in fluid flow communication with said first arm (20) at one end and with said first chamber (17) at its other end,
said second opening (18) being in direct communication with a low pressure side of each of said first water column (32) and said water seal **(22),**
said air flow sensitive buoyant valve (38) being interposed between said third chamber (15) and said first arm (20) of said second chamber (16),
said water seal (22) having a seal opening (53) and being configured in such a manner that a predetermined volume of water in said water seal (22) is sufficient to completely fill up said first arm (20) and to close said valve (38), but not sufficient to maintain continuous closure of said valve (38),
the valve (38) comprising,
a valve chamber (40) having top and bottom portions, said top portion forming an aperture (50) in fluid communication with said third chamber (15), said bottom portion being substantially open to said second chamber (16),
a valve body (46) disposed inside said valve chamber (40), said valve body (46) defining a hollow interior chamber (45) having a top section, said top section including a guide (43) for facilitating engagement of said valve body (46) to said aperture (50),
a seal (44) interposed between said valve body (46), and said aperture (50), said seal (44) closing off fluid flow communication between said second and third chambers (16,15) when said valve body (46) is engaged to said aperture (50),
said valve body (46) being shaped so that when, due to a negative pressure condition in said third chamber (15), a second water column (39) rises to cause said valve body (46) to be seated against said aperture (50) in fluid-tight engagement thereto, and the height of the water level (55) in relation to said seal opening (53) subsequently allows generation of an air pocket, said air pocket subsequently rises through said second water column (39) and engages said valve body (46), thereby momentarily releasing said valve body (46) from its engaged position in a shuttling action between said second water column (39) closing said valve (38) and said air pocket releasing said valve (38) until said negative pressure condition is eliminated.

2. The chest drainage unit (11) according to claim 1, wherein said chest drainage unit (11) has a fourth opening (60) for draining fluids from said third chamber (15).

3. The chest drainage unit (11) according to claim 1, wherein said valve body (46) has a hollow-shaped configuration that is aerodynamically designed to allow the valve (38) to close said aperture (50) from fluid flow communication therethrough in response to a high air flow rate condition in the third chamber (15).

4. The chest drainage unit (11) according to claim 1, wherein said valve body (46) has a hollow-shaped configuration that is aerodynamically designed to allow the valve (38) to close said aperture (50) from fluid flow communication therethrough in response to an extended deep inspiration by a patient (12), when said patient (12) is in fluid flow communication with said chest drainage unit (11) through a tube (14) connected to the third opening (28) of said chest drainage unit (11).

5. The chest drainage unit (11) according to claim 4, wherein said valve (38) remains closed until the patient (12) stops the deep inspiration, said valve (38) thereby operating to maintain substantially the same pressure level in said third chamber (15) as is present within the pleural cavity of said patient (12) during a deep inspiration.

6. The chest drainage unit (11) according to claim 1, wherein said valve chamber (40) further includes an accumulation chamber (41), said accumulation chamber (41) being adapted for receiving any excess water from said second water column(39) as said valve (38) is shuttling between open and closed states.

7. The chest drainage unit (11) according to claim 1, wherein said third chamber (15) is a collection chamber having a bottom portion and an outlet (60), and said collection chamber (15) includes a filtration system (7) for filtering body fluids which comprises a valve cap (62) positioned in fluid flow communication with said outlet (60), said valve cap (62) permitting reflux of fluid from said outlet (60) back into said collection chamber (15).

8. The chest drainage unit (11) according to claim 1, wherein said third chamber (15) is a collection chamber forming a bottom portion, an inlet (28) and an outlet (60), and said collection chamber (15) includes a filtration system (7) for filtering body fluids which comprises a first filter (57) located within said collection chamber (15), said first filter (57) filtering blood that passes through said inlet (28), and a second filter (61) located adjacent said bottom portion of said collection chamber (15) for refiltering the blood prior to the blood exiting through said outlet (60).

9. The chest drainage unit (11) according to anyone of claims 1 to 8, wherein said first water column (32) and said water seal (22) define during normal operation first and second pressure differentials for establishing unidirectional flow from said first and third openings (30, 28) to said second opening (18) while maintaining a desired sub-atmospheric pressure range in said third chamber (15).

## Patentansprüche

1. Eine Brustdrainageeinheit (11) zum Sammeln von Fluiden, bestimmt durch erste, zweite und dritte miteinander kommunizierende Kammern (17,16,15), wobei die Brustdrainageeinheit (11) ausgebildet ist, um zwischen einer Infusionspumpe (13) und einem Patienten (12) in Fluidströmungsverbindung zwischengeschaltet zu werden, wobei die dritte Kammer (15) eine starre Sammelkammer ist, wobei die zweite Kammer (16) eine U-förmige Wasserdichtungskammer ist und wobei die erste Kammer (17) eine Manometer- oder eine Saugkontrollkammer ist,
wobei die Brustdrainageeinheit (11) erste, zweite und dritte Öffnungen (30,18,28) zum jeweiligen Kommunizieren mit der Atmosphäre, einer Vakuumquelle oder einer Fluidsammelröhre (14) aufweist,
eine erste Wassersäule (32) in der ersten Kammer (17) enthalten ist,
wobei die zweite Kammer (16) einen ersten Arm (20) und einen zweiten Arm (21), eine Wasserdichtung (22) und ein luftströmungsempfindliches, schwimmfähiges Ventil (38) aufweist,
wobei der erste Arm (20) in Fluidströmungsverbindung mit dem zweiten Arm (21) durch die Wasserdichtung (22) am einen Ende und der dritten Kammer (15) am anderen Ende ist,
wobei der zweite Arm (21) in Fluidströmungsverbindung mit dem ersten Arm (20) an einem Ende und mit der ersten Kammer (17) an seinem anderen Ende ist,
wobei die zweite Öffnung (18) in direkter Verbindung mit einer Niedrigdruckseite von sowohl der ersten Wassersäule (32) als auch der Wasserdichtung (22) ist,
wobei das luftströmungsempfindliche, schwimmfähige Ventil (38) zwischen der dritten Kammer (15) und dem ersten Arm (20) der zweiten Kammer (16) zwischengeschaltet ist,
wobei die Wasserdichtung (22) eine Dichtungsöffnung (53) aufweist und derart ausgestaltet ist, dass ein vorgegebenes Wasservolumen in der Wasserdichtung (22) ausreichend ist, um den ersten Arm (20) vollständig aufzufüllen und das Ventil (38) zu schliessen, aber nicht ausreichend, um einen ständigen Verschluss des Ventils (38) aufrechtzuerhalten,
wobei das Ventil (38) umfasst,
eine Ventilkammer (40) mit oberen und unteren Teilen, wobei der obere Teil eine Öffnung (50) in Fluidverbindung mit der dritten Kammer (15) bildet, wobei der untere Teil im wesentlichen offen gegenüber der zweiten Kammer (16) ist,
einen in der Ventilkammer (40) angeordneten Ventilkörper (46), wobei der Ventilkörper (46) eine hohle innere Kammer (45) mit einem oberen Abschnitt bestimmt, wobei der obere Abschnitt eine Führung (43) umfasst, um das Eingreifen des Ventilkörpers (46) in die Öffnung (50) zu erleichtern,
eine Dichtung (44) zwischengeschaltet zwischen dem Ventilkörper (46) und der Öffnung (50), wobei die Dichtung (44) die Fluidströmungsverbindung zwischen der zweiten und der dritten Kammer (16,15) verschliesst wenn der Ventilkörper (46) in die Öffnung (50) eingreift,
wobei der Ventilkörper (46) so ausgestaltet ist, dass infolge einer negativen Druckbedingung in der dritten Kammer (15), eine zweite Wassersäule (39) ansteigt, die bewirkt, dass der Ventilkörper (46) in fluiddichtem Eingriff an die Öffnung (50) gesetzt wird, und die Höhe des Wasserstandes (55) in Beziehung zu der Dichtungsöffnung (53) anschliessend die Bildung eines Lufteinschlusses erlaubt, wobei der Lufteinschluss anschliessend durch die zweite Wassersäule (39) aufsteigt und auf den Ventilkörper (46) auftrifft und dadurch den Ventilkörper (46) vorübergehend von dessen Eingriffstellung durch eine Pendelbewegung zwischen der zweiten Wassersäule (39), welche das Ventil (39) schliesst, und dem Lufteinschluss, welcher das Ventil (38) öffnet, freigibt, bis das negative Druckverhältnis beseitigt ist.

2. Die Brustdrainageeinheit (11) gemäss Anspruch 1, wobei die Brustdrainageeinheit (11) eine vierte Öffnung (60) zur Drainage von Fluiden aus der dritten Kammer (15) aufweist.

3. Die Brustdrainageeinheit (11) gemäss Anspruch 1, wobei der Ventilkörper (46) eine hohl ausgeformte Gestalt aufweist, welche aerodynamisch gestaltet ist, um zu erlauben, dass das Ventil (38) die Öffnung (50) in Antwort auf einen hohen Luftdurchsatzzustand in der dritten Kammer (15) von der Fluidströmungsverbindung durch diese abschliesst.

4. Die Brustdrainageeinheit (11) gemäss Anspruch 1, wobei der Ventilkörper (46) eine hohl ausgeformte Struktur aufweist, welche aerodynamisch gestaltet ist, um zu erlauben, dass das Ventil (38) die Öffnung (50) von der Fluidströmungsverbindung durch diese in Antwort auf eine ausgedehnte, tiefe Einatmung durch einen Patienten (12) abschliesst, wenn der Patient mit der Brustdrainageeinheit (11) mittels eines Schlauches (14), welcher mit der dritten Öffnung (28) der Brustdrainageeinheit (11) verbunden ist, in Fluidströmungsverbindung steht.

5. Die Brustdrainageeinheit (11) gemäss Anspruch 4, wobei das Ventil (38) geschlossen bleibt bis der Patient (12) die tiefe Einatmung beendet, wobei Ventil (38) dabei so arbeitet, dass in der dritten Kammer (15) im wesentlichen das gleiche Druckniveau aufrechterhalten wird, wie es innerhalb der Bauchhöhle des Patienten (12) während einer tiefen Einatmung vorhanden ist.

6. Die Brustdrainageeinheit (11) gemäss Anspruch 1, wobei die Ventilkammer (40) zusätzlich eine Speicherkammer (41) aufweist, wobei die Speicherkammer (41) ausgebildet ist, um jedes überschüssige Wasser von der zweiten Wassersäule (39) aufzunehmen, währenddem das Ventil (38) am pendeln zwischen offenen und geschlossenen Zuständen ist.

7. Die Brustdrainageeinheit (11) gemäss Anspruch 1, wobei die dritte Kammer (15) eine Sammelkammer mit einem unteren Teil und einem Auslass (60) ist und wobei die Sammelkammer (15) ein Filtrationssystem (7) zur Filtrierung von Körperfluiden aufweist, welches einen in Fluidströmungsverbindung mit dem Auslass (60) angeordeten Ventilaufsatz (62) einschliesst, wobei der Ventilaufsatz (62) den Rückfluss von Fluid von dem Auslass (60) zurück in die Sammelkammer (15) erlaubt.

8. Die Brustdrainageeinheit (11) gemäss Anspruch 1, wobei die dritte Kammer (15) eine Sammelkammer ist, welche einen unteren Teil, einen Einlass (28) und einen Auslass (60) bildet, und wobei die Sammelkammer (15) ein Filtrationssystem (7) zur Filtrierung von Körperfluiden einschliesst, welches einen innerhalb der Sammelkammer (15) angeordneten ersten Filter (57) umfasst, wobei der erste Filter (57) Blut, welches durch den Einlass (28) fliesst, filtert, und einen neben dem unteren Teil der Sammelkammer (15) angeordneten zweiten Filter (61), um das Blut wieder zu filtrieren bevor das Blut durch den Auslass (60) austritt.

9. Die Brustdrainageeinheit (11) gemäss einem der Ansprüche 1 bis 8, wobei die erste Wassersäule (32) und die Wasserdichtung (22) während des üblichen Betriebes erste und zweite Druckunterschiede definieren, um eine in eine Richtung verlaufende Strömung von der ersten und dritten Öffnung (30,28) zur zweiten Öffnung (18) zu erstellen, wobei ein gewünschter Unterdruckbereich in der dritten Kammer (15) aufrechterhalten wird.

## Revendications

1. Unité de drainage thoracique (11) pour le prélèvement de fluides, définissant une première, une deuxième et une troisième chambres intercommunicantes (17, 16, 15), ladite unité de drainage thoracique (11) étant adaptée pour être intercalée entre, et dans la communication d'écoulement de fluide avec, une pompe à perfusion (13) et un patient (12), ladite troisième chambre (15) étant une chambre de prélèvement rigide, ladite deuxième chambre (16) étant une chambre à eau en forme de U, et ladite première chambre (17) étant un manomètre ou une chambre de commande d'aspiration, ladite unité de drainage thoracique (11) ayant une première, une deuxième et une troisième ouvertures (30, 18, 28) pour communiquer avec l'atmosphère, une source d'aspiration, et un tube de prélèvement de fluide (14), respectivement, une première colonne d'eau (32) étant incluse dans ladite première chambre (17),
ladite deuxième chambre (16) comprenant une première potence (20) et une deuxième potence (21), un joint hydraulique (22), et une soupape flottante sensible au courant aérien (38),
ladite première potence (20) communiquant par écoulement de fluide avec ladite deuxième potence (21) à travers ledit joint hydraulique (22) à une extrémité et avec ladite troisième chambre (15) à l'autre extrémité,
ladite deuxième potence (21) communiquant par écoulement de fluide avec ladite première potence (20) à une extrémité et avec ladite première chambre (17) à l'autre extrémité,
ladite seconde ouverture (18) étant en communication directe avec un côté basse pression de chacun de ladite première colonne d'eau (32) et dudit joint hydraulique (22),
ladite soupape flottante sensible au courant aérien (38) étant intercalée entre ladite troisième chambre (15) et ladite première potence (20) de ladite deuxième chambre (16),
ledit joint hydraulique (22) ayant une ouverture de joint (53) et étant configuré de telle sorte qu'un volume prédéterminé d'eau dans ledit joint hydraulique (22) soit suffisant pour remplir entièrement ladite première potence (20) et pour fermer ladite soupape (38), mais insuffisant pour maintenir une fermeture constante de ladite soupape (38),
la soupape (38) comprenant,
une chambre de soupape (40) ayant des parties supérieure et inférieure, ladite partie supérieure formant une ouverture (50) en communication fluide avec ladite troisième chambre (15), ladite partie inférieure étant sensiblement ouverte sur ladite deuxième chambre (16),
un corps de soupape (46) disposé à l'intérieur de ladite chambre de soupape (40), ledit corps de soupape (46) définissant une chambre intérieure creuse (45) ayant une section supérieure, ladite section supérieure comprenant un guide (43) pour faciliter l'engagement dudit corps de soupape (46) dans ladite ouverture (50),
un joint (44) intercalé entre ledit corps de soupape (46) et ladite ouverture (50), ledit joint (44) fermant la communication de l'écoulement de fluide entre lesdites deuxième et troisième chambres (16, 15) lorsque ledit corps de soupape (46) est engagé dans ladite ouverture (50),
ledit corps de soupape (46) étant formé de sorte que lorsque, en raison d'une condition de pression négative dans ladite troisième chambre (15), une deuxième colonne d'eau (39) s'élève pour amener ledit corps de soupape (46) en butée sur ladite ouverture (50) dans un engagement étanche à cet endroit, et la hauteur du niveau d'eau (55) en relation avec ladite ouverture de joint (53) permet ensuite de générer une poche d'air, ladite poche d'air s'élève ensuite dans ladite deuxième colonne d'eau (39) et s'engage avec ledit corps de soupape (46), libérant ainsi momentanément ledit corps de soupape (46) de sa position engagée dans une action de navette entre ladite deuxième colonne d'eau (39) fermant ladite soupape (38) et ladite poche d'air libérant ladite soupape (38) jusqu'à ce que ladite condition de pression négative soit éliminée.

2. Unité de drainage thoracique (11) selon la revendication 1, dans laquelle ladite unité de drainage thoracique (11) a une quatrième ouverture (60) pour les fluides de drainage de ladite troisième chambre (15).

3. Unité de drainage thoracique (11) selon la revendication 1, dans laquelle ledit corps de soupape (46) a une configuration de forme creuse qui est conçue aérodynamiquement pour permettre à la soupape (38) de fermer ladite ouverture (50) de la communication d'écoulement de fluide à travers elle, en réponse à une condition de débit volumétrique d'air élevé dans la troisième chambre (15).

4. Unité de drainage thoracique (11) selon la revendication 1, dans laquelle ledit corps de soupape (46) a une configuration de forme creuse qui est conçue aérodynamiquement pour permettre à la soupape (38) de fermer ladite ouverture (50) de la communication d'écoulement de fluide à travers elle, en réponse à une inspiration profonde prolongée d'un patient (12), lorsque ledit patient (12) est en communication d'écoulement de fluide avec ladite unité de drainage thoracique (11) à travers un tube (14) relié à la troisième ouverture (28) de ladite unité de drainage thoracique (11).

5. Unité de drainage thoracique (11) selon la revendication 4, dans laquelle ladite soupape (38) reste fermée jusqu'à ce que le patient (12) cesse l'inspiration profonde, ladite soupape (38) fonctionnant alors pour maintenir sensiblement le même niveau de pression dans ladite troisième chambre (15) que celui qui est présent dans la cavité pleurale dudit patient (12) pendant une inspiration profonde.

6. Unité de drainage thoracique (11) selon la revendication 1, dans laquelle ladite chambre de soupape (40) comprend en outre une chambre d'accumulation (41), ladite chambre d'accumulation (41) étant adaptée pour recevoir tout excès d'eau de ladite deuxième colonne d'eau (39) lorsque ladite soupape (38) fait la navette entre les états ouvert et fermé.

7. Unité de drainage thoracique (11) selon la revendication 1, dans laquelle ladite troisième chambre (15) est une chambre de prélèvement ayant une partie inférieure et un orifice de sortie (60), et ladite chambre de prélèvement (15) comprend un système de filtrage (7) pour filtrer les liquides corporels, qui comprend un bouchon de soupape (62) positionné dans la communication d'écoulement de fluide avec ledit orifice de sortie (60), ledit bouchon de soupape (62) permettant le reflux du fluide dudit orifice de sortie (60) dans ladite chambre de prélèvement (15).

8. Unité de drainage thoracique (11) selon la revendication 1, dans laquelle ladite troisième chambre (15) est une chambre de prélèvement formant une partie inférieure, un orifice d'entrée (28) et un orifice de sortie (60), et ladite chambre de prélèvement (15) comprend un système de filtrage (7) pour filtrer les liquides corporels, qui comprend un premier filtre (57) situé à l'intérieur de ladite chambre de prélèvement (15), ledit premier filtre (57) filtrant le sang qui passe à travers ledit orifice d'entrée (28), et un second filtre (61) situé à côté de ladite partie inférieure (15) pour refiltrer le sang avant la sortie du sang à travers ledit orifice de sortie (60).

9. Unité de drainage thoracique (11) selon l'une quelconque des revendications 1 à 8, dans laquelle ladite première colonne d'eau (32) et ledit joint hydraulique (22) définissent pendant une opération normale un premier et un deuxième différentiels de pression pour établir un écoulement unidirectionnel desdites première et troisième ouvertures (30, 28) vers ladite deuxième ouverture (18), tout en maintenant un intervalle de pression sub-atmosphérique souhaité dans ladite troisième chambre (15).
